# EUROPEAN PATENT SPECIFICATION

(11) **EP 1 456 414 B1**
(45) Date of publication and mention of the grant of the patent: **16.07.2008**
(21) Application number: 02793254.0
(22) Date of filing: 24.12.2002
(51) Int. Cl.: C12Q 1/68

(54) **DNA DETECTION USING PRIMERS HYBRIDIZING TO NON-FRAGMENTATED DNA**
DNA DETEKTION UNTER VERWENDUNG VON PRIMERN, WELCHE MIT NICHT-FRAGMENTIERTER DNA HYBRIDISIEREN
PROCEDE DE DETECTION UTILISANT DES AMORCES HYBRIDISANT AVEC ADN NON-FRAGMENTEE

(30) Priority: 24.12.2001 GB 0130821
(43) Date of publication of application: 15.09.2004
(73) Proprietor: Tayside Health Board, Dundee DD3 8EA (GB)
(72) Inventor: Dow, Eleanor, Cupar, Fife KY14 6HP (GB); Deegan, Patrick, Cambridge CB4 1UD (GB)
(74) Representative: Leathley, Anna Elisabeth
(86) International application number: PCT/GB2002/005918
(87) International publication number: WO 2003/060156

(56) References cited:
- WO-A-02/00930
- WO-A-94/11531
- WO-A-97/07239
- US-A- 5 773 649
- PELLESTOR F ET AL.: "Interphasic analysis of aeuploidy in cancer cell lines using primed in situ labeling" CANCER GENETICS AND CYTOGENETICS, vol. 111, 1999, pages 111-118, XP002252614
- MCKIE A B ET A.: "Alu-Polymerase chain reaction genomic fingerprinting technique identifies multiple genetic loci associated with pancreatic tumourigenesis" GENES, CHROMOSOMES & CANCER, vol. 18, 1997, pages 30-41, XP008020978

## Description

The present invention provides a generic test for use in the detection of cancer, and in particular colorectal cancer. In particular there is provided a method of detecting the presence of non-apoptotic DNA in clinical samples, such as faecal samples.

Cancer is thought to arise through genetic mutations which result in uncontrolled and unregulated cell growth.

Colorectal cancer is one of the most common forms of malignancy in the Western world. Colorectal cancers typically originate in the colorectal epithelium. The development from the initial stages to full blown metastatic cancer can take up to 10 years. The early detection and removal (by surgery) of the tumour can therefore result in prevention of the disease.

Unfortunately colorectal cancer is not generally diagnosed until the tumour is at an advanced stage, at which point the case is generally too severe to be successfully treated by surgery.

Many of the current methods used to diagnose the presence of colorectal cancer are invasive and exhibit poor specificity with the occurrence of a high number of false positive and negative tests. Further, the sensitivity of many of these tests, many of which are assays, can be low.

Such problems are compounded by the fact that during the early stages of the cancer, only a small amount of cellular material from the cancerous cells is available and thus, reliable detection of this cellular material can be further problematic.

US 5773649 describes a method of diagnosis and prognosis for tumour cells focussing or polymorphic loci so as to distinguish between tumour and non-tumour cells.

WO 9707239 discloses methods of extracting DNA from tumour cells and is particular, from foecal samples.

When undergoing programmed cell death (apoptosis), the DNA of the cell is broken down into small fragments. Apoptosis does not however occur in cancerous cells, such as the tumour cells of colorectal cancer which have been shed from the gut epithelial lining. DNA fragmentation does not occur in cancerous cells, and the DNA remains unfragmented.

Studies of patients with confirmed cases of colorectal cancer indicate that mutations occur at varying sites in the DNA. As the points in the DNA at which the mutations occur vary from individual to individual there is therefore no ubiquitous marker specific to, or indicative of the presence of colorectal tumour cells.

The present invention relates to the unexpected finding that the presence of specific fragments of DNA can be indicative of the presence of cancerous cells and further that the amplification of these fragments can serve to allow them to be used in the diagnosis of cancer. The identification of such fragments will allow diagnosis irrespective of the point of DNA mutation which causes the tumour.

According to the present invention there is provided a method of detecting the presence of cancerous cells in a biological sample, the method including the steps of;
- Providing a biological sample and extracting the DNA therefrom,
- amplifying the extracted DNA using the polymerase chain reaction using primers specific for regions of DNA which are human specific and which are not fragmented during apoptosis,
- subjecting fragments produced by PCR to separation by electrophoresis, and
- detecting the presence of bands indicative of non-fragmented DNA which is derived from cancerous cells.

Preferably the biological sample is a faecal sample.

Alternatively the biological sample is urine, bile or cerebrospinal fluid or is obtained from any other body fluid where non-cancerous human cells are absent.

Preferably the faecal sample includes any material, including fluid which is derived or extracted from stool.

Preferably the method can be used to screen for the presence of cancerous cells derived from the gut epithelium.

Preferably the primers selected for use in the polymerase chain reaction amplify sequences of DNA consisting of alphoid repeat regions.

Alternatively the primers selected for use in the polymerase chain reaction amplify sequences of DNA consisting of ALU repeat regions.

The present invention further provides a method of detecting the presence of colorectal cancer, the method including the steps of;
- taking a faecal sample and extracting the DNA therefrom,
- amplifying the extracted DNA using the polymerase chain reaction using primers specific for regions of DNA which are human specific and which are not fragmented during apoptosis,
- subjecting fragments produced by PCR to separation by electrophoresis, and
- detecting the presence of banding indicative of non-fragmented DNA which is derived from cancerous cells.

Preferably the faecal sample includes any material, including fluid which is derived or extracted from stool.

Preferably the method can be used to screen for the presence of cancerous cells derived from the gut epithelium.

Preferably the primers selected for use in the polymerase chain reaction amplify sequences of DNA consisting of alphoid repeat regions.

Alternatively the primers selected for use in the polymerase chain reaction amplify sequences of DNA consisting of ALU repeat regions.

A yet further embodiment of the present invention provides a method of detecting the presence of cancerous cells in a biological sample, the method including the steps of;
- providing a biological sample and extracting the DNA therefrom,
- using polymerase chain reaction and specific primers to amplify the alphoid repeat regions located in the extracted DNA,
- subjecting fragments produced by PCR to separation by electrophoresis, and
- detecting the presence of banding indicative of DNA which has not fragmented and which is derived from cancerous cells.

Preferably the biological sample is a faecal sample.

Alternatively the biological sample is urine, bile or cerebrospinal fluid or is obtained from any other body fluid where non-cancerous human cells are absent.

Preferably the faecal sample includes any material, including fluid which is derived or extracted from stool.

Preferably the method can be used to screen for the presence of cancerous cells derived from the gut epithelium.

Preferably the primers selected for use in the polymerase chain reaction amplify sequences of DNA consisting of alphoid repeat regions.

Alternatively the primers selected for use in the polymerase chain reaction amplify sequences of DNA consisting of ALU repeat regions.

A method of detecting the presence of cancerous cells in a biological sample, the method including the steps of;
- providing a biological sample and extracting the DNA therefrom,
- using polymerase chain reaction and specific primers to amplify the repeat regions located between ALU repeats in the extracted DNA,
- subjecting fragments produced by PCR to separation by electrophoresis, and
- detecting the presence of banding indicative of DNA which has not fragmented and which is derived from cancerous cells.

Preferably the biological sample is a faecal sample.

Alternatively the biological sample is urine, bile or cerebrospinal fluid or is obtained from any other body fluid where non-cancerous human cells are absent.

Preferably the faecal sample includes any material, including fluid which is derived or extracted from stool.

Preferably the method can be used to screen for the presence of cancerous cells derived from the gut epithelium.

Preferably the primers selected for use in the polymerase chain reaction amplify sequences of DNA consisting of alphoid repeat regions.

Alternatively the primers selected for use in the polymerase chain reaction amplify sequences of DNA consisting of ALU repeat regions.

The present invention will now be described, by way of example only, with reference to the accompanying drawings wherein;
Figure 1 shows banding resolved on a gel following separation of DNA fragments by electrophoresis, the DNA having been extracted from a faecal sample and amplified using PCR primers selected to bind regions containing alphoid repeats, and
Figure 2 shows an example of a gel following the amplification by PCR and running out on a gel of DNA extracted from a faecal sample wherein the repeats between the ALU repeats have been amplified.

Alphoid sequences are repeat sequences found near the centrosomes on chromosomes. These alphoid repeats are generally around 170 bp in length and make up around 1% of human DNA and consist of up to a dozen evenly spaced repeats. Upon selection of appropriate primers, it is possible to generate a range of products, depending upon whether amplification has terminated at the first, second or subsequent repeats.

The (non-apoptotic) cancerous cells which are shed from a tumour can have their DNA extracted and further can be run out on a size resolving gel in order to show the presence of a ladder of bands. This laddering relates to alphoid repeat sequences which are amplified from the DNA of the cancerous cell, this being possible as the DNA in the cell is non-fragmented as it has not undergone apoptosis.

Specifically for the detection of colorectal cancer, a.faecal sample is obtained and the DNA is extracted therefrom. DNA extraction may be effected by any suitable method known in the art. Cancerous cells will be present in the stool sample due to them being sloughed off of the epithelial wall.

The DNA extracted from these faecal samples is then amplified using polymerase chain reaction (PCR). Primers are selected which will amplify the alphoid repeat regions.

As mentioned above, the PCR will produce a number of bands of differing sizes, the size being dependent on where amplification terminates.

The product of the PCR is subsequently run out on a size resolving gel in order to show the different band lengths, this difference in length being indicative of a differing number of alphoid repeats in the band.

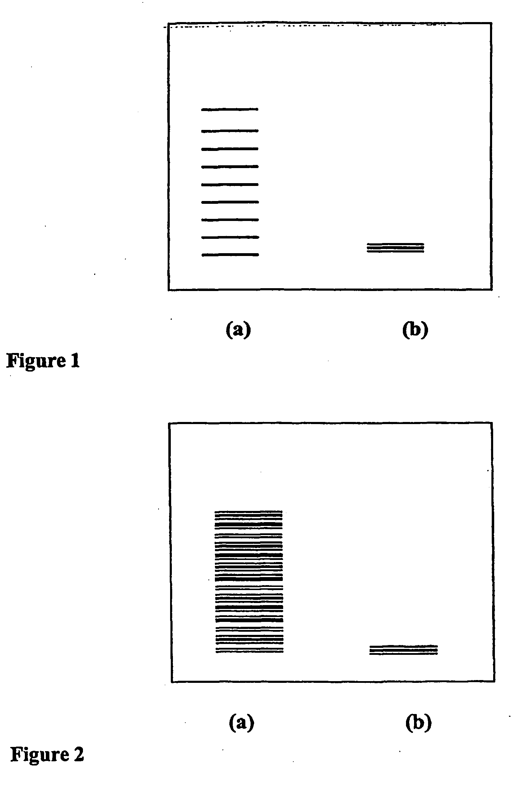

Figure 1 shows an example of non-apoptotic DNA banding (labelled a) run out on a size resolving gel, following the extraction and amplification of the DNA.

The distinct "laddering" like banding indicative of the presence of alphoid sequences can be seen. The bands (labelled b) show DNA from non-cancerous cells which have undergone apoptosis and thus where there has been DNA fragmentation.

The detection of the ladder of bands on the gel indicates the presence of non-apoptotic cells in the sample. If the sample has been taken from a body fluid which does not normally contain live cells, then the assumption can be made that the non apoptotic cells are cancerous.

Detection of these ladder like bands is potentially automatable and may be applied to detect any tumour from samples into which live (non-apoptotic) cancerous cells would be shed or present.

Further, although the specific example of extracting DNA from a faecal sample is described above, it would also be possible to extract and amplify DNA from other body fluid samples such as bile, urine and cerebrospinal fluid or sample or material, including fluid, derived or extracted from stool, or any other material derived from the aerodigestive tract or any other suitable body fluid which does not contain living human cells.

The use of these alternative sample medium would allow the present invention to be used to detect cancer types other than colorectal cancer.

In an alternative embodiment of the present invention, the DNA extracted from a faecal (or other) sample can be screened for the presence of ALU repeat sequences.

ALU repeat sequences occur at random positions within the DNA. In unfragmented DNA, this will lead to a plurality of bands of varying lengths being resolved by electrophoresis on a size-separating gel.

In non-cancerous cells, where the DNA is fragmented, only short lengths of DNA are present, and accordingly the DNA is resolved by the gel to give rise to bands of relatively short length.

The extent of DNA fragmentation in non-apoptotic cells is such that no short repeats are seen on amplification, as the fragmentation of the DNA produces bands which are too short to be detected.

Figure 2 shows an example of a gel exhibiting such banding. The bands in column a representing the plurality of bands resolved for and indicative of the presence of unfragmented DNA. Column b shows bands of relatively short length where DNA fragmentation has occurred.

In some instances, the degree of DNA fragmentation is such that only very small fragments of DNA are present. These small fragments could in some cases run off the gel during electrophoresis. In such situations it may be difficult to establish whether the absence of banding is due to there being no DNA present in the sample or alternatively due to fragmentation being so extensive that the small fragments have run off the gel.

This is therefore problematic in that a control for the test is not always evident. In such cases, alternative methods should be employed to establish whether the absence of such banding is due to there being no DNA in the sample, or alternatively because no tumour is present and thus that there is no unfragmented DNA in the sample.

The present invention therefore provides for a new method of screening for cancer which is particularly advantageous as it does not rely on the identification of a specific point mutation as an indicator of cancer. Further, the technique can be completely non-invasive, and thus has associated benefits to both the patient and the overall cost of screening.

The screening method can therefore be widely used across the population to detect cancer, and in particular, colorectal cancer.

The present invention exhibits the further advantage that a diagnosis of colorectal cancer can be made without knowledge of specific DNA cancer markers being required.

The screening method of the present invention produces results which allow for a greater accuracy in diagnosis and thus accordingly result in more appropriate referrals for further clinical investigation in suspected cases of cancer. This in turn minimises costs in carrying out exploratory procedures where not required, and also minimises inconvenience to patients whom have been mis-diagnosed with the condition.

Although the invention has been particularly shown and described with reference to a preferred embodiment, it will be understood by those skilled in the art that various changes in the form and details may be made therein without departing from the scope of the invention.

## Claims

1. A method of detecting the presence of cancerous cells in a biological sample through the determination of the presence of non-apoptotic DNA, the method including the steps of:
- providing DNA extracted from a biological sample,
- amplifying the extracted DNA using the polymerase chain reaction using primers directed to alphoid repeat sequences or ALU repeat regions, of human DNA,
- subjecting fragments produced by PCR to separation by electrophoresis, and
- detecting the presence or absence of bands indicative of non-fragmented DNA which is indicative of non-apoptotic cells.

2. The method of claim 1, further comprising the step of providing a biological sample and extracting the DNA therefrom.

3. The method of claim 1 or 2 wherein the biological sample is a faecal sample, urine, bile or cerebrospinal fluid or is obtained from any other body fluid where non-cancerous human cells are absent.

4. The method of any one of claims 1 to 3 wherein the faecal sample includes any material, including fluid which is derived or extracted from stool.

5. The method of claim 4 wherein the method can be used to screen for the presence of cancerous cells derived from the gut epithelium.

6. A method of detecting the presence of colorectal cancer in a biological sample through the determination of the presence of non-apoptotic DNA, the method including the steps of:
- taking DNA extracted from a faecal sample,
- amplifying the extracted DNA using the polymerase chain reaction using primers specific for alphoid repeat sequences or ALU repeat regions of human DNA,
- subjecting fragments produced by PCR to separation by electrophoresis, and
- detecting the presence or absence of banding indicative of non-fragmented DNA which is indicative of non-apoptotic cells.

7. The method of claim 6 further comprising the step of taking a faecal sample and extracting the DNA therefrom.

8. The method of claim 6 or 7 wherein the biological sample is a faecal sample, urine, bile or cerebrospinal fluid or is obtained from any other body fluid where non-cancerous human cells are absent.

9. The method of any one of claims 6 to 8 wherein the faecal sample includes any material, including fluid which is derived or extracted from stool.

10. The method of claim 9 wherein the method can be used to screen for the presence of cancerous cells derived from the gut epithelium.

11. Use of primers specific for alphoid repeat sequences to detect non-apoptotic DNA in a method for the detection of cancer in a biological sample.

12. Use of primers specific for ALU repeat regions to detect non-apoptotic DNA in a method for the detection of cancer in a biological sample.

## Patentansprüche

1. Verfahren zur Detektion des Vorliegens krebsartiger Zellen in einer biologischen Probe über die Bestimmung des Vorliegens nicht-apoptotischer DNA, wobei man bei diesem Verfahren
- aus einer biologischen Probe extrahierte DNA bereitstellt,
- die extrahierte DNA unter Verwendung der Polymerase-Kettenreaktion und von Primern, die gegen Alphoid-Repeat-Sequenzen oder ALU-Repeat-Regionen menschlicher DNA gerichtet sind, amplifiziert,
- mit den über PCR hergestellten Fragmenten eine Trennung über Elektrophorese durchführt, und
- das Vorliegen oder Fehlen von Banden detektiert, die auf nicht-fragmentierte DNA hinweisen, welche auf nicht-apoptotische Zellen hinweist.

2. Verfahren nach Anspruch 1, wobei man in einem weiteren Schritt eine biologische Probe bereitstellt und die DNA daraus extrahiert.

3. Verfahren nach Anspruch 1 oder 2, wobei die biologische Probe eine Fäces-Probe, Urin, Gallenflüssigkeit oder Cerebrospinalflüssigkeit ist oder aus einer beliebigen anderen Körperflüssigkeit erhalten wird, in der nicht-krebsartige menschliche Zellen fehlen.

4. Verfahren nach einem der Ansprüche 1 bis 3, wobei die Fäces-Probe beliebiges Material umfasst, einschließlich Flüssigkeit, die aus Stuhl stammt oder extrahiert ist.

5. Verfahren nach Anspruch 4, wobei das Verfahren zum Screenen auf das Vorliegen von krebsartigen Zellen verwendet werden kann, die aus dem Darmepithel stammen.

6. Verfahren zur Detektion des Vorliegens von kolorektalem Krebs in einer biologischen Probe über die Bestimmung des Vorliegens nicht-apoptotischer DNA, wobei man bei diesem Verfahren
- aus einer Fäces-Probe extrahierte DNA nimmt,
- die extrahierte DNA unter Verwendung der Polymerase-Kettenreaktion und von Primern, die gegen Alphoid-Repeat-Sequenzen oder ALU-Repeat-Regionen menschlicher DNA gerichtet sind, amplifiziert,
- mit den über PCR hergestellten Fragmenten eine Trennung über Elektrophorese durchführt, und
- das Vorliegen öder Fehlen von Bandenmustern detektiert, die auf nicht-fragmentierte DNA hinweisen, welche auf nicht-apoptotische Zellen hinweist.

7. Verfahren nach Anspruch 6, wobei man in einem weiteren Schritt eine Fäces-Probe nimmt und die DNA daraus extrahiert.

8. Verfahren nach Anspruch 6 oder 7, wobei die biologische Probe eine Fäces-Probe, Urin, Gallenflüssigkeit oder Cerebrospinalflüssigkeit ist oder aus einer beliebigen anderen Körperflüssigkeit erhalten wird, in der nicht-krebsartige menschliche Zellen fehlen.

9. Verfahren nach einem der Ansprüche 6 bis 8, wobei die Fäces-Probe beliebiges Material umfasst, einschließlich Flüssigkeit, die aus Stuhl stammt oder extrahiert ist.

10. Verfahren nach Anspruch 9, wobei das Verfahren zum Screenen auf das Vorliegen von krebsartigen Zellen verwendet werden kann, die aus dem Darmepithel stammen.

11. Verwendung von Primern, die für Alphoid-Repeat-Sequenzen spezifisch sind, zur Detektion nicht-apoptotischer DNA ein einem Verfahren zur Detektion von Krebs in einer biologischen Probe.

12. Verfahren von Primern, die für ALU-Repeat-Regionen spezifisch sind, zur Detektion von nicht-apoptotischer DNA in einem Verfahren zur Detektion von Krebs in einer biologischen Probe.

## Revendications

1. Procédé pour détecter la présence de cellules cancéreuses dans un échantillon biologique par détermination de la présence d'ADN non apoptotique, le procédé comprenant les étapes consistant à :
- fournir de l'ADN extrait d'un échantillon biologique,
- amplifier l'ADN extrait en utilisant la réaction de polymérisation en chaîne utilisant des amorces visant des séquences répétées alphoïdes ou des régions répétées ALU de l'ADN humain,
- soumettre les fragments produits par PCR à la séparation par électrophorèse, et
- détecter la présence ou l'absence de bandes indicatives d'ADN non fragmenté qui est indicatif de cellules non apoptotiques.

2. Procédé selon la revendication 1, comprenant en outre l'étape consistant à fournir un échantillon biologique et à en extraire l'ADN.

3. Procédé selon la revendication 1 ou 2 dans lequel l'échantillon biologique est un échantillon fécal, de l'urine, de la bile ou du liquide céphalorachidien ou est obtenu à partir d'un autre liquide organique quelconque dans lequel il n'y a pas de cellules humaines cancéreuses.

4. Procédé selon l'une quelconque des revendications 1 à 3 dans lequel l'échantillon fécal inclut un matériau quelconque, incluant un fluide qui est dérivé ou extrait de selles.

5. Procédé selon la revendication 4 dans lequel le procédé peut être utilisé pour détecter la présence de cellules cancéreuses dérivées de l'épithélium intestinal.

6. Procédé pour détecter la présence d'un cancer colorectal dans un échantillon biologique par détermination de la présence d'ADN non apoptotique, le procédé incluant les étapes consistant à :
- prendre de l'ADN extrait d'un échantillon fécal,
- amplifier l'ADN extrait en utilisant la réaction de polymérisation en chaîne utilisant des amorces spécifiques de séquences répétées alphoïdes ou de régions répétées ALU de l'ADN humain,
- soumettre les fragments produits par PCR à la séparation par électrophorèse, et
- détecter la présence ou l'absence de formation de bandes indicatives de l'ADN non fragmenté qui est indicatif de cellules non apoptotiques.

7. Procédé selon la revendication 6 comprenant en outre l'étape consistant à prendre un échantillon fécal et à en extraire l'ADN.

8. Procédé selon la revendication 6 ou 7 dans lequel l'échantillon biologique est un échantillon fécal, de l'urine, de la bile ou du liquide céphalorachidien ou est obtenu à partir d'un autre liquide organique quelconque dans lequel il n'y a pas de cellules humaines cancéreuses.

9. Procédé selon l'une quelconque des revendications 6 à 8 dans lequel l'échantillon fécal inclut un matériau quelconque incluant du fluide qui est dérivé ou extrait de selles.

10. Procédé selon la revendication 9, le procédé pouvant être utilisé pour détecter la présence de cellules cancéreuses dérivées de l'épithélium intestinal.

11. Utilisation d'amorces spécifiques de séquences répétées alphoïdes pour détecter de l'ADN non apoptotique dans un procédé pour la détection d'un cancer dans un échantillon biologique.

12. Utilisation d'amorces spécifiques de régions répétées ALU pour détecter de l'ADN non apoptotique dans un procédé pour la détection d'un cancer dans un échantillon biologique.
